(19) Europäisches Patentamt
European Patent Office
Office européen des brevets

(11) **EP 3 151 798 B1**

(12) **EUROPEAN PATENT SPECIFICATION**

(45) Date of publication and mention
of the grant of the patent:
**25.07.2018 Bulletin 2018/30**

(51) Int Cl.:
*A61F 13/15* (2006.01)    *B05D 1/00* (2006.01)
*B32B 7/14* (2006.01)

(21) Application number: **15731768.6**

(22) Date of filing: **03.06.2015**

(86) International application number:
**PCT/US2015/033864**

(87) International publication number:
**WO 2015/187755 (10.12.2015 Gazette 2015/49)**

(54) **METHOD FOR APPLYING ADHESIVES IN PATTERNS TO AN ADVANCING SUBSTRATE**

VERFAHREN ZUM ANBRINGEN VON HAFTSTOFFEN IN MUSTERN AUF EINEM SICH VORSCHIEBENDEN SUBSTRAT

PROCÉDÉ D'APPLICATION D'ADHÉSIFS EN MOTIFS SUR UN SUBSTRAT MOBILE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.06.2014 US 201462007965 P**

(43) Date of publication of application:
**12.04.2017 Bulletin 2017/15**

(73) Proprietor: **The Procter & Gamble Company Cincinnati, OH 45202 (US)**

(72) Inventors:
• **BROWN, Darrell, Ian**
  **Cincinnati, Ohio 45202 (US)**
• **KAWKA, Paul, Anthony**
  **Cincinnati, Ohio 45202 (US)**

(74) Representative: **Mather, Peter Geoffrey**
**Procter & Gamble Service GmbH**
**IP Department**
**Sulzbacher Straße 40-50**
**65824 Schwalbach am Taunus (DE)**

(56) References cited:
**US-A- 5 654 040        US-A1- 2011 274 834**
**US-A1- 2014 148 773        US-A1- 2014 148 774**

## Description

FIELD OF THE INVENTION

The present disclosure relates to methods and apparatuses utilizing continuous substrates for manufacturing articles, and more particularly, methods and apparatuses for applying viscous fluid, such as adhesives, to an advancing substrate.

BACKGROUND OF THE INVENTION

Along an assembly line, various types of articles, such as for example, diapers and other absorbent articles, may be assembled by adding components to and otherwise modifying an advancing, continuous web of material. For example, in some processes, advancing webs of material are combined with other advancing webs of material. In other examples, individual components created from advancing webs of material are combined with advancing webs of material, which in turn, are then combined with other advancing webs of material. Webs of material and component parts used to manufacture diapers may include: backsheets, topsheets, absorbent cores, front and/or back ears, fastener components, and various types of elastic webs and components such as leg elastics, barrier leg cuff elastics, and waist elastics. Once the desired component parts are assembled, the advancing web(s) and component parts are subjected to a final knife cut to separate the web(s) into discrete diapers or other absorbent articles. The discrete diapers or absorbent articles may also then be folded and packaged.

Various methods and apparatuses may be used for attaching different components to the advancing web and/or otherwise modify the advancing web. For example, some production operations are configured to apply relatively high viscosity fluids, such as hot melt adhesives, to an advancing web. In some instances, the production operations are configured to apply hot melt adhesives to an advancing web in pre-determined patterns. These operations may include the use of systems and methods such as slot die coating, direct gravure, offset gravure and reverse gravure roll coating processes that are extensively described in the art. However, current systems and methods for applying patterned adhesives to an advancing substrate may have certain limitations.

Some current systems are configured with apparatuses and methods that apply adhesives and other fluids to a substrate in patterns with relatively high resolution and high speeds without being limited by the speed of on/off cycling of switching valves used to interrupt the flow of fluid to the slot die of the fluid applicator. For example, some systems may include patterned roll positioned adjacent a slot die applicator, wherein the patterned roll may be separated from the slot die applicator by a gap distance. During operation, a substrate having an unconstrained caliper that is greater than the gap distance may be advanced between the patterned roll and the slot die applicator. As the substrate advances between the patterned roll and the slot die applicator fluid is discharged from the slot die applicator onto the substrate in a pattern that corresponds with the pattern on the patterned roll. In some configurations, the ability to achieve fine resolution of on/off patterned discharged of adhesive on an advancing substrate is dependent upon the gap distance relative to the unconstrained caliper of the substrate.

For example, US 2011/274834, published on November 10th 2011, discloses the application of viscous fluids, such as adhesives, in pre-determined patterns to an advancing substrate by means of a slot die applicator.

Consequently, it would be beneficial to provide apparatuses and methods that can monitor and/or control the gap distances between patterned rolls and slot die applicators with relatively high resolution and high speeds while taking into account various system operating conditions.

SUMMARY OF THE INVENTION

Aspects of the present invention involve methods for applying fluids onto an advancing substrate. Fluid application apparatus are discussed below in the context of applying adhesives to an advancing substrate having an unconstrained caliper, Hs, and having a first surface disposed opposite of a second surface. The fluid application apparatus may include a slot die applicator and a substrate carrier. The slot die applicator may include a slot opening, a first lip, and a second lip, the slot opening located between the first lip and the second lip. And the substrate carrier may be adapted to advance the substrate past the slot die applicator as the slot die applicator discharges adhesive onto the substrate. In operation, when the first surface of the substrate is disposed on the substrate carrier, the substrate carrier advances the second surface of the substrate past the slot opening of the slot die applicator. The substrate carrier may include a base surface and a pattern element. And the pattern element includes a pattern surface and protrudes outward from the base surface. The substrate carrier is positioned adjacent the slot die applicator to define a minimum distance, Hg, between the pattern surface of the pattern element and the first lip and the second lip that is less than the unconstrained caliper, Hs, of the substrate.

According to the present invention a method for applying a fluid to a substrate in a pattern comprises the steps of: providing a slot die applicator comprising a slot opening, a first lip, and a second lip, the slot opening located between the first lip and the second lip; providing a substrate carrier comprising a pattern element, wherein the pattern element comprises a pattern surface; positioning the slot die applicator adjacent the substrate carrier to define a minimum distance, Hg, between the pattern surface of the pattern element and the first lip and the second lip; advancing the substrate to the substrate carrier, the substrate having a first surface disposed opposite of a second surface and an unconstrained caliper, Hs, wherein the unconstrained caliper, Hs, of the substrate is greater than minimum distance, Hg; advancing the second surface of the substrate past the slot die applicator while the first surface of the substrate is disposed on the substrate carrier; discharging fluid from the slot opening of the slot die applicator onto the second surface of the substrate in a pattern area having a shape that corresponds with a shape of the pattern surface on the substrate carrier by advancing the pattern surface of the pattern element past the first lip, the slot opening, and the second lip of the slot die applicator while the first surface of the substrate is disposed on the substrate carrier; and moving the first lip and the second lip of the slot die applicator either away from or toward the pattern surface to maintain the minimum distance, Hg, between the pattern surface of the pattern element and the first lip and the second lip; and sensing a temperature, T, of the slot die applicator.

BRIEF DESCRIPTION OF THE DRAWINGS

Figure 1 is a perspective view of a fluid application apparatus positioned adjacent to an advancing substrate.

Figure 2A is a perspective view of an embodiment of a substrate carrier including a pattern roller having a continuous base surface and a plurality of pattern surfaces.

Figure 2B is a detailed cross-sectional view of the substrate carrier shown in Figure 2A taken along the line 2B-2B.

Figure 2C is a top side view of a substrate showing a first example adhesive pattern thereon.

Figure 3A is a perspective view of an embodiment of a substrate carrier including a pattern roller having a continuous pattern surface and plurality of base surfaces.

Figure 3B is a detailed cross-sectional view of the substrate carrier shown in Figure 3A taken along the line 3B-3B.

Figure 3C is a top side view of a substrate showing a second example adhesive pattern thereon.

Figure 4 is a schematic cross-sectional side view of an example substrate carrier.

Figure 4A1 is a detailed view of the substrate carrier of Figure 4 including a compliant pattern element and a compliant base layer connected with a base roll.

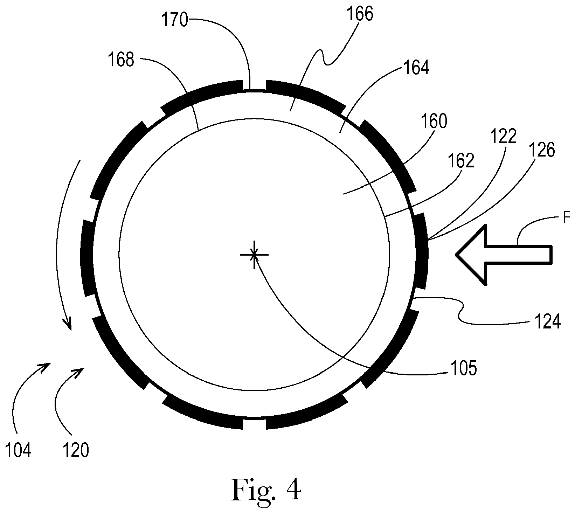

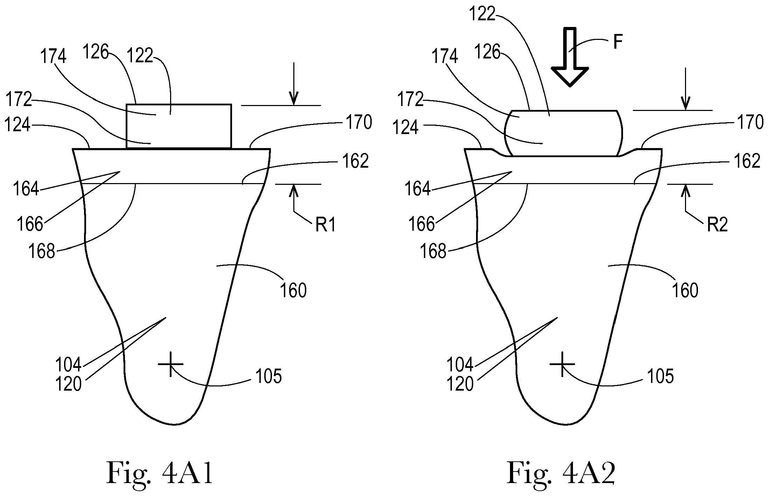

Figure 4A2 is a detailed view of the pattern surface of the pattern element from Figure 4A1 deflected by a force or forces applied to the pattern surface.

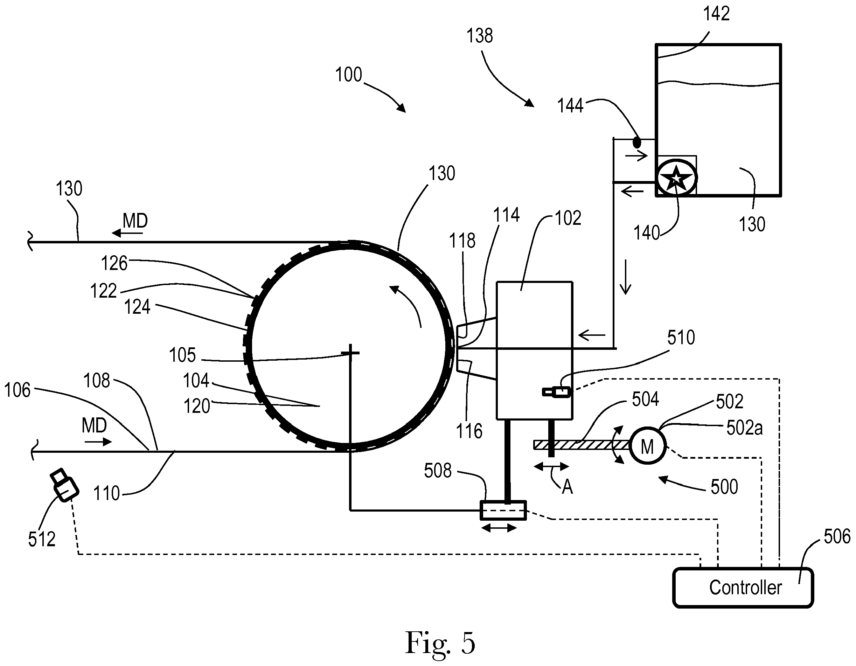

Figure 5 is a schematic cross-sectional side view of a fluid application apparatus.

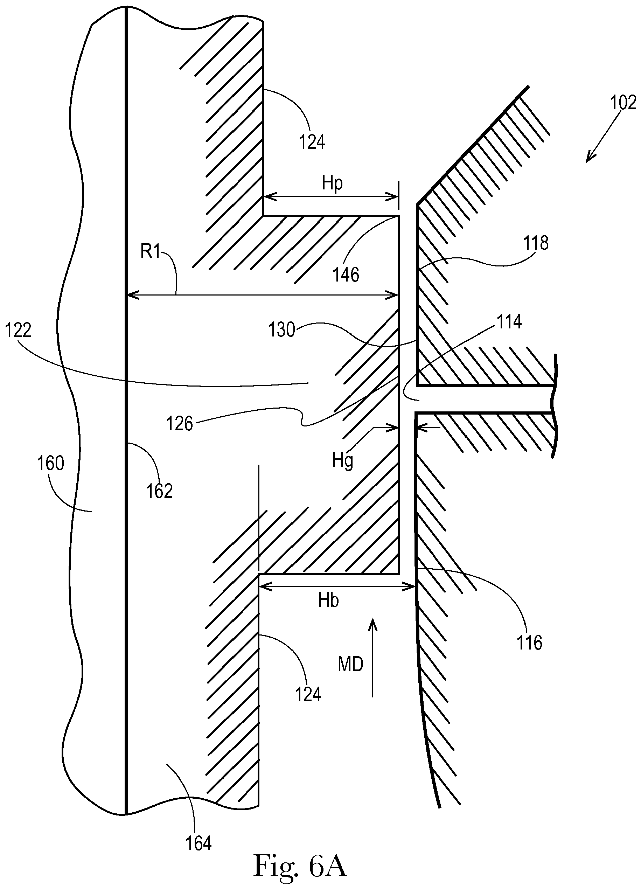

Figure 6A is a detailed cross-sectional view of the substrate carrier of Figure 5 without the substrate wherein the pattern surface of a pattern element is adjacent a first lip, a second lip, and slot opening of the slot die applicator.

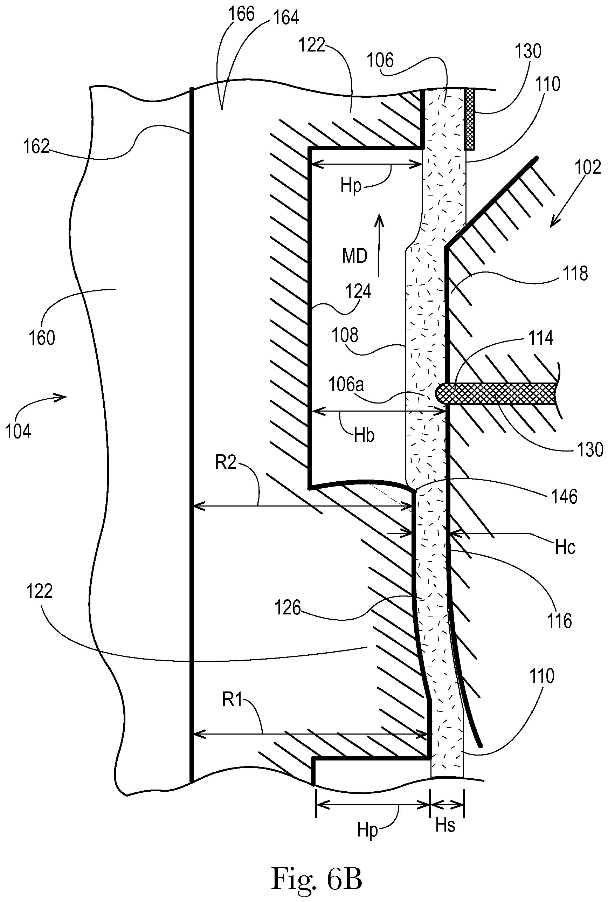

Figure 6B is a detailed cross-sectional view of a substrate carrier and a substrate advancing past a slot die applicator and showing the substrate between a slot opening of the slot die applicator and an advancing base surface.

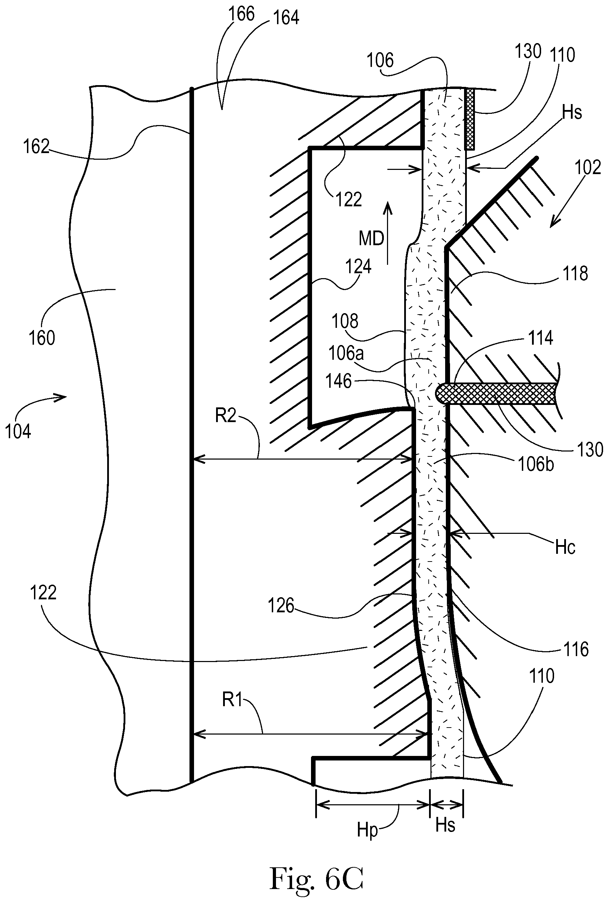

Figure 6C is a detailed cross-sectional view of the substrate carrier and substrate of Figure 6B wherein the base surface is advancing past the slot opening of the slot die applicator such that the substrate is between the slot opening of the slot die applicator and a leading edge of an advancing pattern surface.

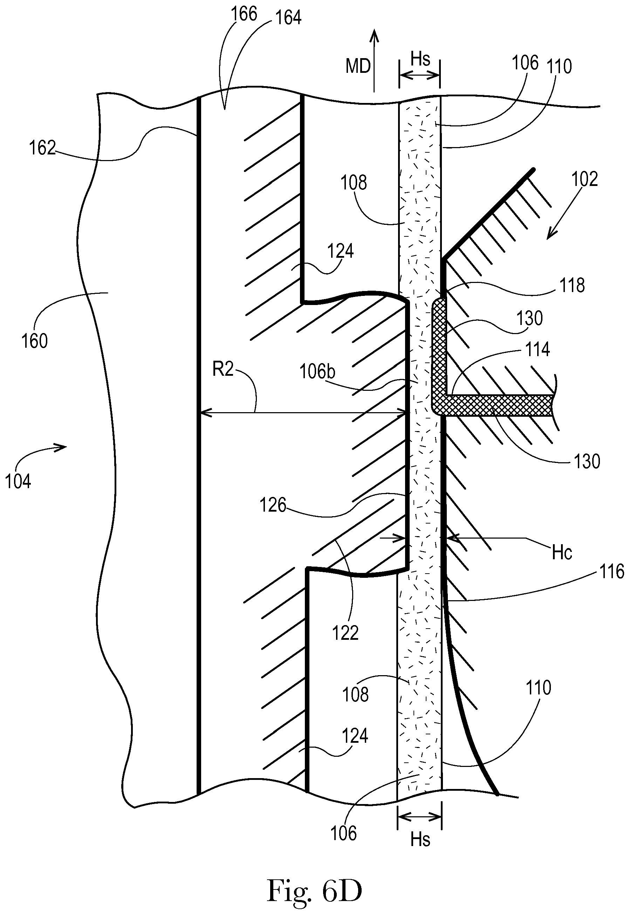

Figure 6D is a detailed cross-sectional view of the substrate carrier and substrate of Figure 6C wherein the base surface has advanced past the slot opening of the slot die applicator such that the substrate is between the slot opening of the slot die applicator and an advancing pattern surface.

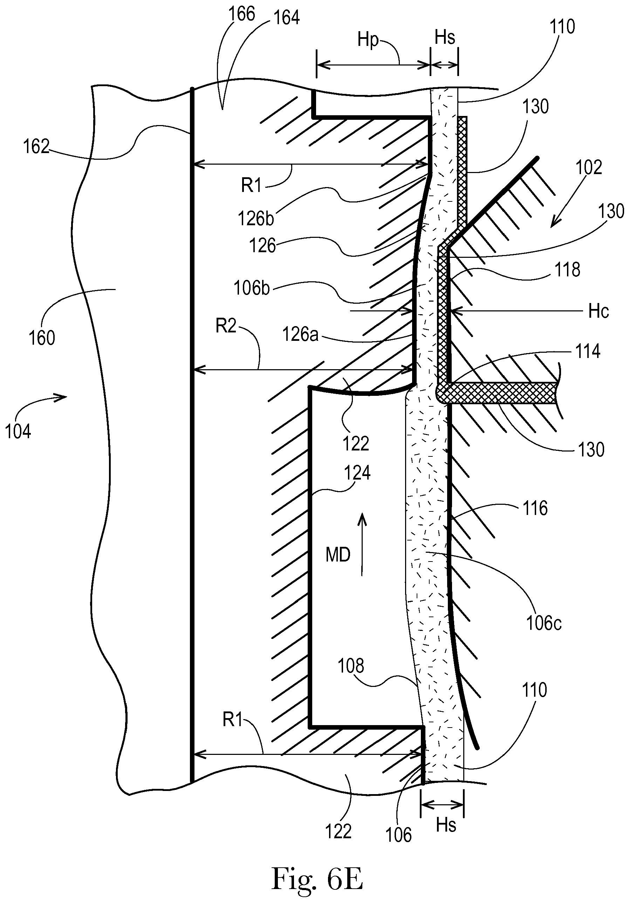

Figure 6E is a detailed cross-sectional view of the substrate carrier and substrate of Figure 6D wherein the pattern surface has advanced past the slot opening of the slot die applicator.

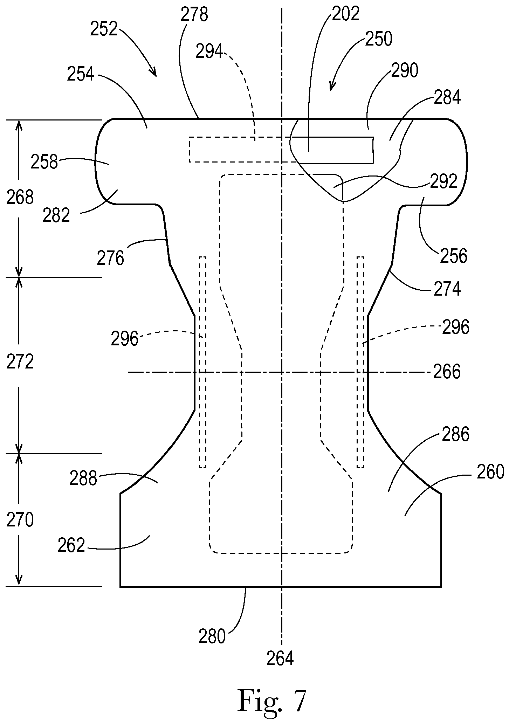

Figure 7 is a top plan view of a disposable absorbent article.

DETAILED DESCRIPTION OF THE INVENTION

The following term explanations may be useful in understanding the present disclosure:

"Absorbent article" is used herein to refer to consumer products whose primary function is to absorb and retain soils and wastes. Non-limiting examples of absorbent articles include diapers, training pants, pull-on pant-type diapers, refastenable diapers or pant-type diapers, incontinence briefs and undergarments, diaper holders and liners, feminine hygiene garments such as panty liners, absorbent inserts, and the like.

"Diaper" is used herein to refer to an absorbent article generally worn by infants and incontinent persons about the lower torso.

The term "disposable" is used herein to describe absorbent articles which generally are not intended to be laundered or otherwise restored or reused as an absorbent article (e.g., they are intended to be discarded after a single use and may also be configured to be recycled, composted or otherwise disposed of in an environmentally compatible manner).

The term "disposed" is used herein to mean that an element(s) is formed (joined and positioned) in a particular place or position as a macro-unitary structure with other elements or as a separate element joined to another element.

As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.

The term "substrate" is used herein to describe a material which is primarily two-dimensional (i.e. in an XY plane) and whose thickness (in a Z direction) is relatively small (i.e. 1/10 or less) in comparison to its length (in an X direction) and width (in a Y direction). Non-limiting examples of substrates include a layer or layers or fibrous materials, films and foils such as plastic films or metallic foils that may be used alone or laminated to one or more web, layer, film and/or foil. As such, a web is a substrate.

The term "nonwoven" refers herein to a material made from continuous (long) filaments (fibers) and/or discontinuous (short) filaments (fibers) by processes such as spunbonding, meltblowing, and the like. Nonwovens do not have a woven or knitted filament pattern.

The term "machine direction" (MD) is used herein to refer to the direction of material flow through a process. In addition, relative placement and movement of material can be described as flowing in the machine direction through a process from upstream in the process to downstream in the process.

The term "cross direction" (CD) is used herein to refer to a direction that is generally perpendicular to the machine direction.

The terms "elastic" and "elastomeric" as used herein refer to any material that upon application of a biasing force, can stretch to an elongated length of at least about 110% of its relaxed, original length (i.e. can stretch to 10% more than its original length), without rupture or breakage, and upon release of the applied force, recovers at least about 40% of its elongation. For example, a material that has an initial length of 100 mm can extend at least to 110 mm, and upon removal of the force would retract to a length of 106 mm (40% recovery). The term "inelastic" refers herein to any material that does not fall within the definition of "elastic" above.

The term "extensible" as used herein refers to any material that upon application of a biasing force, can stretch to an elongated length of at least about 110% of its relaxed, original length (i.e. can stretch to 10 %), without rupture or breakage, and upon release of the applied force, shows little recovery, less than about 40% of its elongation.

The terms "activating", "activation" or "mechanical activation" refer to the process of making a substrate, or an elastomeric laminate more extensible than it was prior to the process.

"Live Stretch" includes stretching elastic and bonding the stretched elastic to a substrate. After bonding, the stretched elastic is released causing it to contract, resulting in a "corrugated" substrate. The corrugated substrate can stretch as the corrugated portion is pulled to about the point that the substrate reaches at least one original flat dimension. However, if the substrate is also elastic, then the substrate can stretch beyond the relaxed length of the substrate prior to bonding with the elastic. The elastic is stretched at least 25% of its relaxed length when it is bonded to the substrate.

As used herein, the term "unconstrained caliper" refers to the caliper of the substrate measured according to Edana WSP 120.1 (05), with a circular presser foot having a diameter of $25.40 \pm 0.02$ mm and an applied force of 2.1 N (i.e. a pressure of $4.14 \pm 0.21$ kPa is applied).

As used herein, the term "compliant" refers to any material with a durometer hardness of 90 or less as measured according to ASTM International Designation: D2240-05 (Reapproved 2010) for Type M durometers.

As used herein, the term "non-compliant" refers to any material with a hardness value greater than 100 HRBW as defined on the Rockwell B Scale in the American National Standard Designation.

Aspects of the present disclosure involve methods and apparatuses utilizing continuous substrates for manufacturing articles, and more particularly, methods and apparatuses for applying fluids onto an advancing substrate. Particular embodiments of the apparatuses and methods disclosed herein provide for the application of viscous fluids, such as adhesives, and in some embodiments, the application of adhesives in pre-determined patterns to an advancing substrate. Embodiments of a fluid application apparatus are discussed in more detail below in the context of applying adhesives to an advancing substrate having an unconstrained caliper, Hs, and having a first surface disposed opposite of a second surface. The fluid application apparatus may include a slot die applicator and a substrate carrier. The slot die applicator may include a slot opening, a first lip, and a second lip, the slot opening located between the first lip and the second lip. And the substrate carrier may be adapted to advance the substrate past the slot die applicator as the slot die applicator discharges adhesive onto the substrate. In operation, when the first surface of the substrate is disposed on the substrate carrier, the substrate carrier advances the second surface of the substrate past the slot opening of the slot die applicator. It is to be appreciated that the apparatus and processes disclosed herein may be used to apply various types of fluids and adhesives in various different patterns to an advancing substrate other than those described and depicted herein.

As discussed in more detail below, the substrate carrier may include a base surface and a pattern element. The pattern element includes a pattern surface and protrudes outward from the base surface. As such, in substrate carriers configured with a base surface, the pattern surface and the base surface are separated by a distance, Hp. In addition, the substrate carrier is positioned adjacent the slot die applicator to define a minimum distance, Hg, between the pattern surface of the pattern element and the first lip and the second lip that is less than the unconstrained caliper, Hs, of the substrate, wherein a sum of the distance, Hp, and distance, Hg, is greater than the unconstrained caliper, Hs, of the substrate. Thus, as the substrate carrier advances the second surface of the substrate past the slot opening, the pattern element is advanced such that the pattern surface repeatedly advances past the first lip, the slot opening, and the second lip of the slot die applicator. In turn, the substrate is intermittently compressed between the slot die applicator and the pattern surface of the pattern element. As the substrate is intermittently compressed, adhesive discharged from the slot die applicator is applied onto the second surface of the advancing substrate in an area having a shape that is substantially the same as a shape defined by the pattern surface. In some embodiments, the pattern element and/or the base surface of the substrate carrier may also be compliant or compressible. And as such, the pattern element and/or the base surface of the substrate carrier may be intermittently compressed as the substrate advances between the slot die applicator and the pattern surface. As such, the pattern surface of the pattern element may deflect away from the slot die applicator as the substrate and the pattern element advance past the first lip, the slot opening, and the second lip of the slot die applicator. And as the pattern surface is intermittently deflected away from the slot die applicator, adhesive discharged from the slot die applicator is applied onto the second surface of the advancing substrate. As mentioned above, the adhesive is thus applied to the substrate in an area having a shape that is substantially the same as a shape defined by the pattern surface.

Based on the foregoing operational description, it is to be appreciated that a desired minimum distance, Hg, between the pattern surface of the pattern element and the first lip and the second lip may be established based on various operating parameters. Such operating parameters may include, for example, the type of substrate material; the unconstrained caliper, Hs, of the substrate; the type fluid to be discharged from the slot die applicator; the substrate carrier material; and/or pattern shape. Once the desired minimum distance, Hg, between the pattern surface of the pattern element and the first lip and the second lip is established, it may be desired to monitor; maintain; and/or change the desired minimum distance, Hg, between the pattern surface of the pattern element and the first lip and the second lip during operation. As discussed in more detail below, embodiments of apparatuses for applying fluids onto an advancing substrate may be configured to monitor; establish; maintain; and/or change the desired minimum distance, Hg, before and/or during operation. For example, the slot die applicator and/or the substrate carrier may be adapted to move relative to each other. In some embodiments, the slot die applicator may be connected with a drive mechanism configured to move the slot die applicator away from and toward the substrate carrier. As such, the drive mechanism may be configured to move slot die applicator back and forth to establish; maintain; and/or change the desired minimum distance, Hg, before and/or during operation. In some embodiments, the drive mechanism may include one or more motors connected indirectly or directly with the slot die applicator. As discussed in more detail below, the drive mechanism may be configured to move the slot die applicator based on various parameters, such as for example, a temperature, T, of the slot die applicator; motor feedback device counts; torque of the motor, and/or a measured distance, Md, between the pattern surface of the pattern element and the first lip and the second lip.

The apparatuses and methods disclosed herein may include substrate carriers having various configurations, such as disclosed for example in U.S. Patent No. 8,186,296 and U.S. Patent Publication Nos. 2014/0148774A1; 2014/0144579A1; 2014/0148323A1; and 2014/0148773A1. For example, in some embodiments the substrate carrier may be configured as a roller. In other embodiments, the substrate carrier may include an endless belt. The substrate carriers may also utilize various outer surface arrangements. For example, the base surface may be configured as a continuous surface and the substrate carrier may include a plurality of discrete pattern elements separated from each other by the continuous surface. In such a configuration, each pattern element may include a pattern surface and each pattern element may protrude outward from the continuous surface such that each pattern surface is separated from the continuous surface by the distance, Hp. In another example, the pattern surface may be configured as a continuous surface and the base surface may include a plurality of discrete base surfaces separated from each other by the pattern element. In such a configuration, the pattern element may protrude outward from each of the base surfaces such that each base surface is separated from the continuous surface by the distance, Hp. It is to be appreciated that the pattern surface of the pattern element may be configured in various different shapes and sizes and may be configured to define various different patterns. As such, adhesive may be transferred from the slot die applicator to define various patterns on a substrate.

As mentioned above, apparatuses and methods of the present disclosure may be utilized to apply adhesives to continuous substrates used in the manufacture of absorbent articles. Such substrates may be utilized in absorbent article components such as, for example: backsheets, topsheets, absorbent cores, front and/or back ears, fastener components, and various types of elastic webs and components such as leg elastics, barrier leg cuff elastics, and waist elastics. Exemplary descriptions of absorbent article components and substrates are provided below with reference to Figure 7. In addition, substrates may include continuous webs of material and component parts mounted on carrier substrates or may be in the form of a continuous substrate.

Although much of the present disclosure is provided in the context of manufacturing absorbent articles, it is to be appreciated that the apparatuses and methods disclosed herein may be applied to the manufacture of other types of articles and products manufactured from continuous substrates. Examples of other products include absorbent articles for inanimate surfaces such as consumer products whose primary function is to absorb and retain soils and wastes that may be solid or liquid and which are removed from inanimate surfaces such as floors, objects, furniture and the like. Non-limiting examples of absorbent articles for inanimate surfaces include dusting sheets, pre-moistened wipes or pads, pre-moistened cloths, paper towels, dryer sheets and dry-cleaning clothes such. Additional examples of products include absorbent articles for animate surfaces whose primary function is to absorb and contain body exudates and, more specifically, devices which are placed against or in proximity to the body of the user to absorb and contain the various exudates discharged from the body. Non-limiting examples of incontinent absorbent articles include diapers, training and pull-on pants, adult incontinence briefs and undergarments, feminine hygiene garments such as panty liners, absorbent inserts, and the like, toilet paper, tissue paper, facial wipes or clothes, and toilet training wipes. Still other examples of products may include packaging components and substrates and/or containers for laundry detergent and coffee, which may be produced in pellets or pouches and may be manufactured in a converting or web process or even discrete products produced at high speed such as high-speed bottling lines, cosmetics, razor blade cartridges, and disposable consumer batteries.

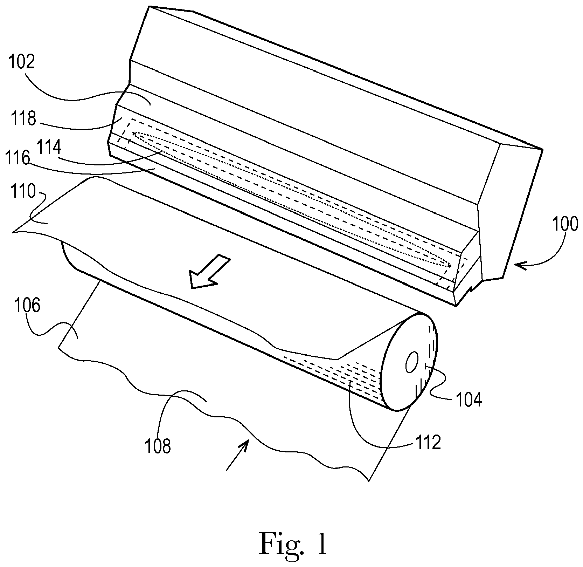

Figure 1 shows a perspective view an embodiment of an apparatus 100 for applying adhesives to a substrate. The apparatus 100 includes a slot die applicator 102 and a substrate carrier 104. As shown in Figure 1, a substrate 106 is advancing in a machine direction and is partially wrapped around the substrate carrier 104. More particularly, the substrate 106 includes a first surface 108 disposed opposite a second surface 110. And the first surface 108 of the substrate 106 is disposed on an outer surface 112 of the substrate carrier 104 while the second surface 110 of the substrate 106 advances past the slot die applicator 102. As discussed in more detail below, the second surface 110 of the substrate 106 advances past the slot die applicator 102 and adhesive is transferred from the slot die applicator 102 onto the second surface of the substrate in a pattern that is substantially the same as a pattern defined on the outer surface 112 of the substrate carrier 104. As discussed in more detail below, the substrate carrier 104 may be configured in various ways to deposit fluid 130 discharged from a slot die applicator 102 onto a substrate 106 in various different patterns.

It is to be appreciated that the slot die applicator 102 shown in Figure 1 is a generic representation of a device that is used to apply adhesive to the substrate 106. The slot die applicator may include a slot opening 114, a first lip 116, and a second lip 118. The first lip 116 may also be referred to herein as an upstream die lip, and the second lip 118 may also be referred to herein as a downstream die lip. The slot opening 114 is located between the first lip 116 and the second lip 118. Adhesive or other fluid may be discharged from the slot opening 114 onto the second surface 110 of the substrate 106 as the substrate carrier 104 advances the substrate past the first lip 116, slot opening 114, and second lip 118 of the slot die applicator 102. As discussed in more detail below, the substrate 106 is also intermittently compressed between the slot die applicator 102 and substrate carrier 104 as the substrate 106 advances past the slot die applicator 102. It is to be appreciated that various forms of slot die applicators may be used herein to apply adhesive or other fluids to an advancing substrate according to methods and apparatuses. For example, U.S. Patent No. 7,056,386 provides a description of slot die applicators that may be used. Other examples of commercially available slot die applicators include Nordson Corporation's EP11 Series of Slot Die Applicators and ITW Dynatec Gmbh's APEX Series of Slot Die Auto Adhesive Applicators.

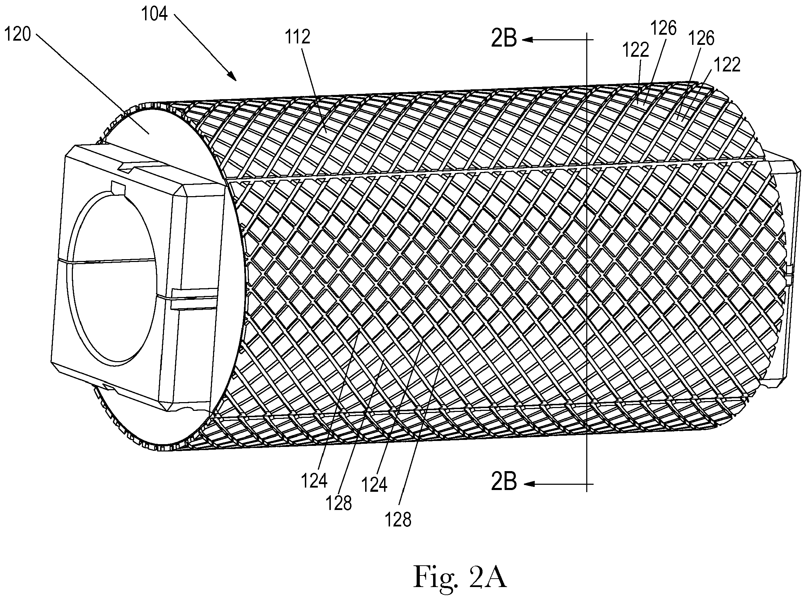

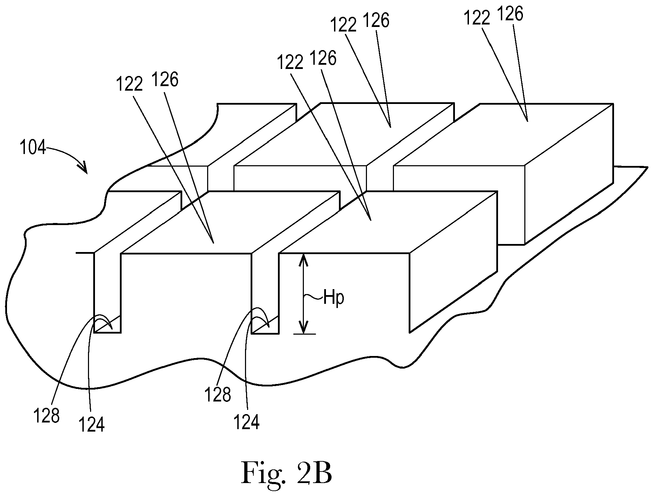

Various types of substrate carriers 104 may be used in accordance with the apparatuses and methods herein. For example, Figures 2A and 2B show an embodiment of a substrate carrier 104 configured as a roller 120 adapted to advance a substrate 106 past the slot die applicator 102. The outer surface 112 of the substrate carrier 104 shown in Figures 2A and 2B includes a plurality of pattern elements 122 that protrude radially outward from a base surface 124. Each pattern element 122 includes a pattern surface 126, and the radial protrusion of the pattern elements 122 from the base surface 124 define a distance, Hp, between the pattern surface 126 and the base surface 124. As shown in Figure 2A and 2B, the base surface 124 is configured as a continuous surface 128, and the plurality of discrete pattern elements 122 are separated from each other by the continuous surface 128. The pattern surfaces 126 in Figures 2A and 2B define a diamond shape. In some embodiments, the shape and size of the pattern surface 126 of each pattern element 122 may be identical or substantially identical to each other. It is to be appreciated that the number, size, and shape of some or all the pattern surfaces and/or pattern elements may be different. In addition, the distance, Hp, between the base surface 124 and the pattern surface 126 of the pattern element 122 may be the same or different for some or all of the pattern elements.

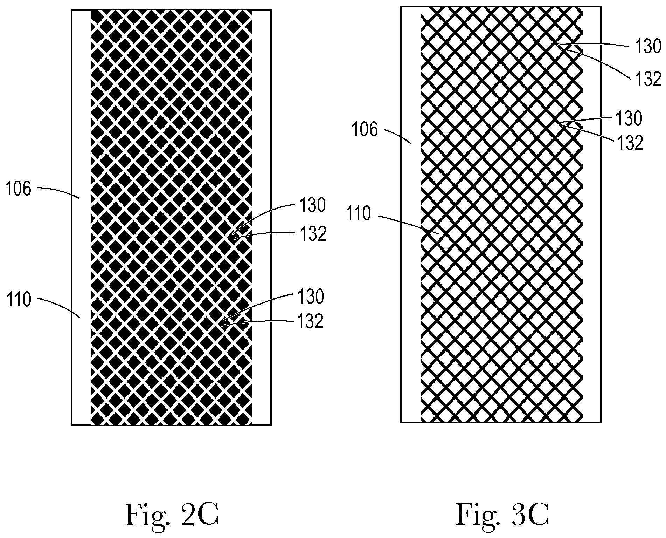

As discussed in more detail below, as the substrate carrier 104 advances the substrate 106 past the slot die applicator 102, fluid discharged from the slot die applicator is deposited onto the substrate in a pattern substantially matching the shapes of the pattern surfaces on the substrate carrier. For example, Figure 2C shows an example pattern of fluid 130 deposited on a second surface 110 of a substrate 106 after being advanced past a slot die applicator while disposed on a substrate carrier having pattern elements 122 and pattern surfaces 126 similar to those shown in Figures 2A and 2B. As shown in Figure 2C, the fluid 130 is deposited onto the substrate 106 in discrete pattern areas 132 having diamond shapes that correspond with and may mirror the shapes of the pattern surfaces 126 on the substrate carrier 104 shown in Figure 2A.

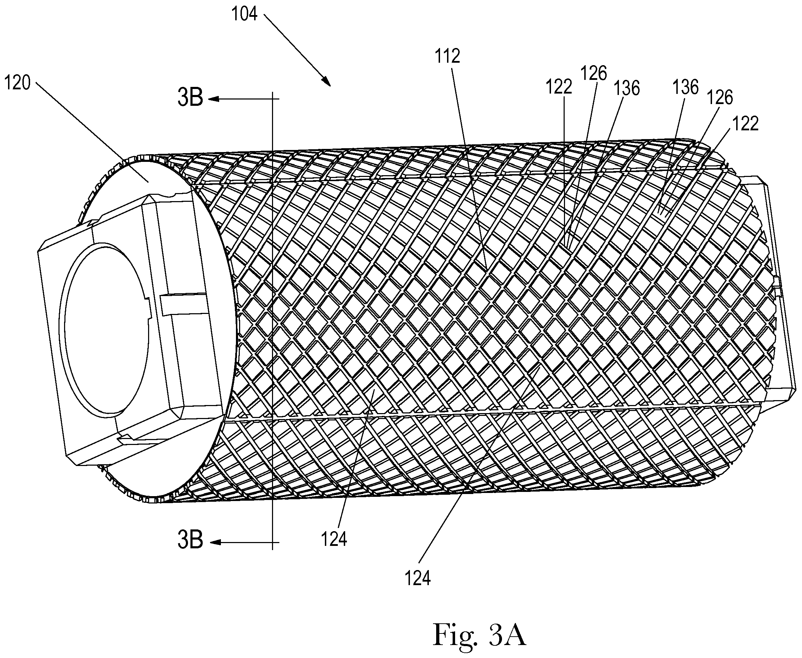

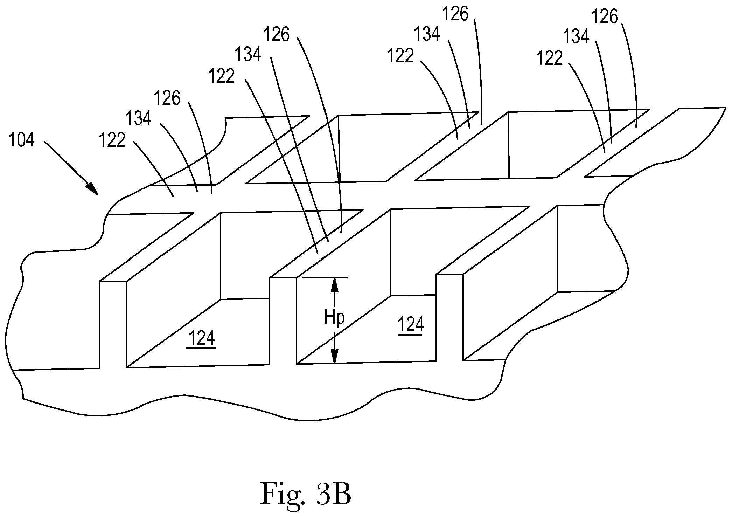

Figures 3A and 3B show another embodiment of a substrate carrier 104 configured as a roller 120 adapted to advance a substrate 106 past the slot die applicator 102. The substrate carrier 104 shown in Figures 3A and 3B includes a single pattern element 122 including a pattern surface 126. And the pattern element 122 protrudes radially outward from a plurality of base surfaces 124. More particularly, the pattern surface 126 is configured as a continuous surface 134 and the plurality of base surfaces are separated from each other by the pattern element 122. The radial protrusion of the pattern element 122 from the base surfaces 124 defines a distance, Hp, between the pattern surface 126 and the base surfaces 124. The pattern surface 126 in Figures 3A and 3B defines a continuous crossing line pattern wherein the shape and size of each base surface 124 are identical or substantially identical to each other. It is to be appreciated that the number, size, and shape of some or all the base surfaces may be different. In addition, the distance, Hp, between the base surfaces 124 and the pattern surface 126 of the pattern element 122 may be the same or different for some or all of the base surfaces. It should also be appreciated that the substrate carrier may be configured without base surfaces. For example, the substrate carrier may include a plurality of holes and the pattern surface may be configured as a continuous surface wherein the plurality of holes are separated from each other by the pattern element.

As previously mentioned, as the substrate carrier 104 advances the substrate 106 past the slot die applicator 102, fluid 130 discharged from the slot die applicator 102 is deposited onto the substrate 106 in a pattern substantially matching the shape of the pattern surface 126 on the substrate carrier 104. For example, Figure 3C shows an example pattern of fluid 130 deposited on a second surface 110 of a substrate 106 after being advanced past a slot die applicator 102 while disposed on a substrate carrier 104 having a pattern element 122 and pattern surface 126 similar to that shown in Figures 3A and 3B. As shown in Figure 3C, the fluid 130 is deposited onto the substrate 106 in a crossing line pattern defining diamond shapes therebetween that correspond with and may mirror the shapes of the base surfaces 124 on the substrate carrier 104 shown in Figures 3A and 3B.

As previously mentioned, the substrate carrier may be constructed in various ways such that the base surface and/or pattern elements may or may not include compliant materials. In some configurations, the compliant material(s) may be compressible to allow a pattern surface of a pattern element to deflect away from the slot die applicator. Thus, the substrate carrier may be configured such that deflection of the pattern surface away from the slot die applicator compresses the pattern element and/or base surface as the substrate and the pattern element advance past the first lip, the slot opening, and the second lip of the slot die applicator.

Figure 4 shows a schematic cross-sectional side view of an example substrate carrier 104 that may be configured with compliant materials and components that can be compressed and allow the pattern surface 126 to deflect in response to a force or forces, F, exerted on the pattern surface 126. The substrate carrier 104 in Figure 4 is in the form of a roller 120 adapted to rotate around an axis of rotation 105. In operation, a force or forces, F, may be exerted on the pattern surface 126 as the substrate 106 and the pattern element 122 advance past the first lip 116, the slot opening 114, and the second lip 118 of the slot die applicator 102. It is to be appreciated that the substrate carrier 104 may be configured in various ways with various different components of compliant materials that allow the pattern surface 126 to deflect.

For example, Figures 4A1 and 4A2 show a detailed view of the substrate carrier 104 in the form of a roller 120, such as from Figure 4, including a compliant pattern element 122 and a compliant base surface 124 connected with a base roll 160 having a non-compliant support surface 162. More particularly, the roller 120 in Figures 4A1 and 4A2 may include a base layer 164 of compliant material extending radially outward from the non-compliant support surface 162 to define the compliant base surface 124. In some arrangements, the base layer 164 of compliant material may be formed as a cylindrically shaped sleeve or tube 166 having an inner radial surface 168 and an outer radial surface 170. The inner radial surface 168 may surround all or a portion of the non-compliant support surface 162 of the base roll 160, and the outer radial surface 170 may define all or a portion of the base surface 124. In turn, the pattern element 122 may include a proximal end portion 172 and a distal end portion 174 that includes the pattern surface 126, wherein the proximal end portion 172 is connected with outer radial surface 170 of the base layer 164. As such, the pattern element 122 may extend radially outward from the base layer 164 of compliant material to the distal end portion 174. It is to be appreciated that the pattern element 122 may be separately connected with or integrally formed with the compliant base layer 164. Figure 4A1 shows the pattern element 122 and base layer 164 of compliant material in an uncompressed state, wherein the minimum distance between the pattern surface 126 and the non-compliant support surface 162 is defined by distance, R1. Figure 4A2 shows the compliant pattern element 122 and compliant base layer 164 of Figure 4A1 in a compressed state wherein a force or forces, F, are applied to the pattern surface 126. Because the pattern element 122 and base layer 164 are both compliant, the force or forces, F, applied to the pattern surface 126 causes the pattern element 122 and the base layer 164 to be compressed against the non-compliant surface 162 of the base roll 160. The compression of the pattern element 122 and the base layer 164 allows the pattern surface 126 to deflect in response to the forces, F. As such, the minimum distance between the pattern surface 126 and the non-compliant surface 162 is defined as distance, R2, wherein R2 is less than R1.

It is to be appreciated that substrate carrier may be configured in various ways other than discussed with reference to Figures 4A1 and 4A2. For example, the substrate carrier 104 may be in the form of a roller 120, such as from Figure 4, including a non-compliant pattern element 122 and a compliant base surface 124 connected with a base roll 160 having a non-compliant support surface 162. In yet another example, the substrate carrier 104 may be in the form of a roller 120 from Figure 4 including a compliant pattern element 122 connected with a base roll 160, wherein the base roll 160 includes a non-compliant outer circumferential support surface 162 that also defines the base surface 124. In yet another example, the substrate carrier 104 may be in the form of a roller 120, such as from Figure 4, including a non-compliant pattern element 122, a compliant base surface 124 connected with a base roll 160 having a non-compliant support surface 162. In such a non-compliant substrate carrier configuration, only the substrate 106 may be compressed between the slot die applicator 102 and the pattern surface 126 of the pattern element 122 as the pattern element advances past the first lip 116, the slot opening 114, and the second lip 118 of the slot die applicator 102.

As previously mentioned, the methods and apparatuses herein include a substrate carrier adapted to advance a substrate past a slot die applicator. Figure 5 shows a schematic cross-sectional side view of an embodiment of a fluid application apparatus 100 including a substrate carrier 104 and a slot die applicator 102. The substrate 106 includes a first surface 108 and a second surface 110 disposed opposite the first surface 108. A portion of the first surface 108 of the substrate 106 is disposed on the substrate carrier 104, which may be configured as a roller 120 having a plurality of pattern elements 122 protruding from a plurality of base surfaces 124. It is to be appreciated that the substrate carrier 104 shown in Figure 5 may be configured with various features and aspects of any substrate carriers discussed herein, including those discussed above with reference to Figures 1 through 4A2. The roller 120 rotates to advance the second surface 110 of the substrate 106 past the slot die applicator 102. A fluid delivery system 138 may be used to supply fluid 130, such as an adhesive, to the slot die applicator 102. It is to be appreciated that the fluid delivery system may be configured in various different ways. For example, as shown in Figure 5, the fluid delivery system 138 may include a pump 140 to move fluid from a tank 142 to the slot die applicator 102. The fluid delivery system 138 may also be configured with a pressure relief valve 144 configured to help control the pressure of the fluid 130 fed from the pump 140. Fluid 130 from the fluid delivery system 138 passes through the slot die applicator 102 and slot opening 114 and is transferred to the second surface 110 of the advancing substrate 106.

With continued reference to Figure 5, fluid 130 passing from the slot die applicator 102 is transferred to the second surface 110 of the substrate 106 in a pattern or shape that is substantially the same as the pattern surfaces 126 on the substrate carrier 104. As discussed in more detail below, the substrate carrier 104 is positioned adjacent the slot die applicator 102 to define a minimum distance between the pattern surface 126 and slot die applicator 102, which is less than the unconstrained caliper of the substrate 106. As such, the pattern element and/or base surface may be compressed to allow the pattern surface 126 of the pattern element to deflect away from the slot die applicator 102 as the substrate 106 and the pattern surface 126 of the pattern element 122 advances past the first lip 116, the slot opening 114, and the second lip 118 of the slot die applicator 102. However, the minimum distance between the base surface 124 of the substrate carrier 104 and the slot die applicator 102 is greater than the unconstrained caliper of the substrate 106. As such, the base surface 124 is not compressed as the substrate advances past the first lip 116, the slot opening 114, and the second lip 118 of the slot die applicator 102. Thus, in operation, although fluid 130 is continuously discharged from the slot die applicator 102, fluid 130 is transferred to the advancing substrate 106 when the pattern element 122 and/or base surface 124 is compressed as pattern surfaces 126 on the substrate carrier 102 advance past the slot die opening 114 and deflect the pattern surface 126. And fluid 130 is not transferred to the advancing substrate 106 when the pattern element 122 and/or base surface 124 are uncompressed while the base surfaces 124 on the substrate carrier 104 advance past the slot die opening 114. The following provides a more detailed description of fluid transfer from the slot die applicator to the substrate with reference to Figures 6A through 6E.

Figure 6A is a detailed cross-sectional view of the substrate carrier of Figure 5 shown without the substrate wherein the pattern surface 126 of a pattern element 122 is adjacent a first lip 116, a second lip 118, and slot opening 114 of the slot die applicator 102. As shown in Figure 6A, the substrate carrier 104 includes a non-compliant support surface 162, a base surface 124, and a pattern element 122 protruding from base surface 124. In an uncompressed state, the pattern element 122 protrudes outward from the base surface 124 to define a distance, Hp, between the pattern surface 126 and the base surface 124, and to define a minimum distance, R1, between the pattern surface 126 and the non-compliant support surface 162. The substrate carrier 104 is also positioned adjacent the slot die applicator 102 to define a minimum distance, Hg, between the pattern surface 126 of the uncompressed pattern element 122 and the first lip 116 and the second lip 118. As discussed below, the minimum distance, Hg, is less than the unconstrained caliper, Hs, of the substrate 106 advanced by the substrate carrier 104. In addition, the substrate carrier 104 is positioned adjacent the slot die applicator 102 to define a minimum distance, Hb, between the base surface 124 and the first lip 116 and the second lip 118. As discussed below, the minimum distance, Hb, may be greater than the unconstrained caliper, Hs, of the substrate advanced by the substrate carrier 104.

Figure 6B is a detailed cross-sectional view of a substrate carrier 104 of Figure 6A and a substrate 106 advancing past a slot die applicator 102. The substrate 106 has an unconstrained caliper, Hs, and has a first surface 108 disposed opposite of a second surface 110. The first surface 108 of the substrate 106 is disposed on the substrate carrier 104. And the substrate 106 and substrate carrier 104 are shown as advancing together in a machine direction, MD, past the slot die applicator 102. More particularly, the second surface 110 of the substrate 106 is advancing past a slot opening 114 located between an upstream lip 116 and a downstream lip 118 of the slot die applicator 102. As previously mentioned, the substrate carrier 104 is positioned adjacent the slot die applicator 102 to define a minimum distance, Hg, between the uncompressed pattern surface 126 of the pattern element 122 and the first lip 116 and the second lip 118 that is less than the unconstrained caliper, Hs, of the substrate 106. In addition, the substrate carrier 104 is positioned adjacent the slot die applicator 102 to define a minimum distance, Hb, between the base surface 124 and the first lip 116 and the second lip 118 that is greater than the unconstrained caliper, Hs, of the substrate. The apparatus 100 may also be configured such that a sum of the distance, Hp, and distance, Hg, is greater than the unconstrained caliper, Hs, of the substrate 106. Thus, a portion 106a of the substrate 106 that is located between the slot opening 114 of the slot die applicator 102 and the advancing base surface 124 is not pressed against the base surface 124. As such, although fluid 130 is continuously discharged from the slot opening 114, fluid 130 is not being transferred to the second surface 110 of the substrate 106.

Figure 6C is a detailed cross-sectional view of the substrate carrier 104 and substrate 106 of Figure 6B wherein the base surface 124 has advanced past the slot opening 114 of the slot die applicator 102 such that a portion 106b of the substrate 106 is between the first lip 116 of the slot die applicator 102 and a leading edge 146 of an advancing pattern surface 126. As previously discussed, the minimum distance, Hg, between the pattern surface 126 of the uncompressed pattern element 122 and the first lip 116 and the second lip 118 is less than the unconstrained caliper, Hs, of the substrate 106. As such, a portion 106b of substrate 106 between the pattern surface 126 and the first lip 116 is pressed against and exerts forces on the pattern surface 126. Thus, the pattern element 122 and/or base surface 124 compresses, allowing the pattern surface 126 to deflect away from the first lip 116 to define a minimum distance, R2, between the pattern surface 126 and the non-compliant support surface 162. The fluid 130 being discharged from the slot opening 114 is shown in Figure 6C as beginning to transfer to the second surface 110 of the substrate as the leading edge 146 of the pattern surface 126 and adjacent portion of the substrate 106 begin to advance past the slot opening 114.

With continued reference to Figure 6C, the compression of the pattern element 122 and/or base surface 124 allows the pattern surface 126 to deflect away from the first lip 116 to define a compressed distance, Hc, between the pattern surface 126 and the first lip 116. When the substrate 106 is made from a material, such as a film, the substrate 106 may maintain a caliper that is substantially the same as the unconstrained caliper, Hs, while advancing between the pattern surface 126 and the first lip 116. Thus, the pattern surface 126 may deflect by a distance represented by the difference of Hg and Hs, and in some instances, the distance R2, may be calculated as:

$$R2 = R1 + Hg - Hs$$

In such a scenario, the compressed distance, Hc, may also be equal to or substantially equal to the unconstrained caliper, Hs.

Still referring to Figure 6C, when the substrate 106 is made from a material, such as a nonwoven or laminate including a nonwoven layer, the substrate 106 may be compressed to a caliper that is less than the unconstrained caliper, Hs, while advancing between the pattern surface 126 and the first lip 116. In such a scenario, the compressed distance, Hc, may be less than the unconstrained caliper, Hs. In other words, the substrate 106 may be compressed to a caliper equal to or substantially equal the compressed distance, Hc. Thus, the pattern surface 126 may deflect by a distance represented by the difference of Hg and Hc, and in some instances, the distance R2, may be calculated as:

$$R2 = R1 + Hg - Hc$$

Figure 6D is a detailed cross-sectional view of the substrate carrier 104 and substrate of Figure 6C wherein the base surface 124 and leading edge 146 of the pattern surface 126 has advanced past the slot opening 114 of the slot die applicator 102 such that the portion 106b of the advancing substrate 106 is between the slot opening 114 of the slot die applicator 102 and an advancing pattern surface 126. Because the minimum distance, Hg, between the pattern surface 126 of the uncompressed pattern element 122 and the first lip 116 and the second lip 118 is less than the unconstrained caliper, Hs, of the substrate 106, the portion 106b of substrate 106 between the pattern surface 126 and the first lip 116 and second lip 118 of the slot die applicator 102 presses against and exerts forces on the pattern surface 126. As such, the compliant pattern element 122 and/or base surface 124 are compressed, allowing the pattern surface 126 to deflect away from the first lip 116 and second lip 118. As mentioned above, when the substrate 106 is made from a material, such as a film, the substrate 106 may maintain a caliper that is substantially the same as the unconstrained caliper, Hs, while advancing between the pattern surface 126 and the first lip 116 and second lip 118. Thus, the pattern surface 126 may deflect by a distance represented by the difference of Hg and Hs, and in some instances, the distance R2, may be calculated as: R2 = R1+Hg-Hs. Also, as mentioned above, when the substrate 106 is made from a material, such as a nonwoven or laminate including a nonwoven layer, the substrate 106 may be compressed to a caliper that is less than the unconstrained caliper, Hs, while advancing between the pattern surface 126 and the first lip 116 and second lip 118. Thus, the pattern surface 126 may deflect by a distance represented by the difference of Hg and Hc, and in some instances, the distance R2, may be calculated as: R2 = R1+Hg-Hc. The fluid 130 being discharged from the slot opening 114 is shown in Figure 6D as being transferred to the second surface 110 of the substrate as the pattern surface 126 and adjacent portion 106b of the substrate 106 advance past the slot opening 114.

Figure 6E is a detailed cross-sectional view of the substrate carrier 104 and substrate 106 of Figure 6D wherein the portion 106b of the substrate and the pattern surface 126 have advanced past the slot opening 114 of the slot die applicator 102. As shown in Figure 6E, an upstream portion 126a of the pattern surface 126 is adjacent the second lip 118, and a downstream portion 126b of the pattern surface 126 has advanced past the second lip 118. As such, the portion 106b of the advancing substrate 106 between the second lip 118 of the slot die applicator 102 and the upstream portion 126a of the advancing pattern surface 126 presses against and exerts forces on the pattern surface 126. As such, the compliant pattern element 122 and/or base surface 124 are compressed, allowing the upstream portion 126a of the pattern surface 126 to deflect away from the first lip 116 and second lip 118 to define the minimum distance, R2, between the upstream portion 126a of the pattern surface 126 and the non-compliant support surface 162.

With continued reference to Figure 6E, the downstream portion 126b of the pattern surface 126 has advanced past the second lip 118 of the slot die applicator 102, and as such, the portion 106b of the substrate 106 is no longer pressing against downstream portion 126b of the pattern surface 126, allowing the compliant pattern element 122 and/or base surface 124 to return to an uncompressed state wherein the downstream portion 126b of the pattern surface 126 deflects back away from the non-compliant surface 162 such that the minimum distance between the non-compliant surface 162 and the downstream portion 126b pattern surface 126 is the distance, R1. Once the upstream portion 126a of the pattern surface 126 has also advanced past the second lip 118, the remainder of the compliant pattern element 122 and/or base surface 124 may return to an uncompressed state wherein the both the upstream portion 126a and downstream portion 126b of the pattern surface 126 have deflected away from the non-compliant surface 162 such that the minimum distance between the non-compliant surface 162 and the pattern surface 126 is the distance, R1.

Still referring to Figure 6E, an uncompressed portion 106c of the advancing substrate 106 is between the slot opening 114 of the slot die applicator 102 and an advancing base surface 124. Because the minimum distance, Hb, between the base surface 124 and the first lip 116 and the second lip 118 that is greater than the unconstrained caliper, Hs, of the substrate, a portion 106c of substrate 106 that advances between the base surface 124, slot opening 114, and the first lip 116 of the slot die applicator 102 is uncompressed. As such, the fluid 130 being discharged from the slot opening 114 is shown in Figure 6E as ceasing to be transferred to the second surface 110 of the substrate 106 as the base surface 124 and adjacent uncompressed portion 106c of the substrate advance past the slot opening 114.

As mentioned above, it is to be appreciated that various forms and configurations of substrate carriers may be used with the presently disclosed methods and apparatuses, such as disclosed for example in U.S. Patent No. 8,186,296 and U.S. Patent Publication Nos. 2014/0148774A1; 2014/0144579A1; 2014/0148323A1; and 2014/0148773A1.

As mentioned above and based on the foregoing operational description, it is to be appreciated that the apparatuses and methods herein may be configured to monitor; establish; maintain; and/or change the desired minimum distance, Hg, between the pattern surface 126 of the uncompressed pattern element 122 and the first lip 116 and the second lip 118 before and/or during operation. As such, the slot die applicator 102 and/or the substrate carrier 104 may be adapted to move relative to each other. For example, as shown in Figure 5, the slot die applicator may be connected with a drive mechanism 500 configured to move the slot die applicator 102 away from and toward the substrate carrier 104 as indicated by the directional arrows "A."

It is to be appreciated that the drive mechanism 500 may be configured in various ways. For example, as shown in Figure 5, the drive mechanism 500 may include a motor 502 connected with slot die applicator 102 through a transmission apparatus 504 that converts the rotational movement of a motor shaft into linear movement, such as indicated by directional arrows "A", of the slot die applicator 102 toward or away from the slot die applicator 104. One example of a transmission apparatus 504 may include a jack-screw mechanism. In other examples, the transmission apparatus 504 may be configured with various arrangements of belts, chains, and/or gears arranged to affect movement of the slot die applicator 102. It is also to be appreciated that drive mechanism 500 may include one or more motors 502 that may be configured in various ways. For example, the motor 502 may be configured as a programmable servo motor that may operate at constant or variable speeds. As shown in Figure 5, the motor 502 may also be connected with a controller 506 that may be configured to control the motor in various ways, such as for example, by varying current supplied to the motor 502. It is to be appreciated that the controller 504 may or may not be integrated into a motor power supply. And the controller 506, along with the motor power supply, may or may not be integrated into the motor 502 itself. In some embodiments, the motor may be configured as programmable linear servo motor that is connected directly with the slot die applicator. It is to be appreciated that the current supplied to the motor 502 can be controlled using any of a variety of a methods for programming motors such as for example, standard cam curve functions, reference data table containing reference points, desired motor encoder points, and the like or combinations thereof. Although the above discussion is provided in the context of imparting movement to the slot die applicator 102, it is to be appreciated that the substrate carrier 104 may be configured to move instead of or in addition to the slot die applicator 102 to establish; maintain; and/or change the desired minimum distance, Hg, before and/or during operation.

It is to be appreciated that the controller 506 may include one or more computer systems. The computer system may, for example, include one or more types of programmable logic controller (PLC), programmable automation controller (PAC), and/or personal computer (PC) running software and adapted to communicate over a network using a protocol. Some embodiments may utilize industrial programmable controllers such as the Siemens S7 series, Rockwell ControlLogix, SLC or PLC 5 series, or Mitsubishi Q series and employ communication protocols including serial communications, DeviceNet, ControlNet, Sercos, TCP/IP, Ethernet/IP, Modbus, ModbusTCP, Profibus, ProfiNet, EtherCAT, I/O Link, and SSCNet. The aforementioned embodiments may use a personal computer or server running a control algorithm such as Rockwell SoftLogix or National Instruments Labview or may be any other device capable of receiving inputs from sensors, performing calculations based on such inputs and generating control actions through servomotor controls, electrical actuators or electro-pneumatic, electrohydraulic, and other actuators. Process and product data may be stored directly in the controller or may be located in a separate data historian. In some embodiments, the historian is a simple data table in the controller. In other embodiments, the historian may be a relational or simple database. Common historian applications include Rockwell Automation Factory Talk Historian, General Electric Proficy Historian, OSI PI, or any custom historian that may be configured from Oracle, SQL or any of a number of database applications. It is also to be appreciated that various types of controllers and inspection sensors can be configured in various ways and with various algorithms to provide various types of data and perform various functions, for example, such as disclosed in U.S. Patent Nos. 5,286,543; 5,359,525; 6,801,828; 6,820,022; 7,123,981; 8,145,343; 8,145,344; and 8,244,393; and European Patent No. EP 1528907B1.

With continued reference to Figure 5, since the slot die applicator 102 is coupled to an output of the motor 502, changes in the angular velocity and position of the motor 502 may directly correlate to changes in position of the slot die applicator 102. Thus, the actual position of the slot die applicator 102 can be detected in various ways. For example, the system may include a motor feedback device 502a that may include a position transducer utilizing an encoder-based system (high resolution absolute encoder or incremental encoder) or resolver-based system that communicates motor feedback counts to the controller 506. In turn, the motor feedback counts may be correlated with the actual position and/or changes in position of the slot die applicator 102. As shown in Figure 5, the apparatus may also include a position sensor 508 to detect the minimum distance, Hg, between the pattern surface 126 of the uncompressed pattern element 122 and the first lip 116 and the second lip 118. Such a sensor 508 may be configured in various ways. For example, in some embodiments the sensor 508 may be configured as a linear variable differential transformer or LVDT. Some embodiments of the sensor 508 may include various forms of measuring devices such as, for example, an inductive sensor, a capacitance sensor, an Eddy-current sensor, a laser triangulation displacement sensor, a confocal-chromatic sensor, and combinations thereof, such as disclosed in U.S. Patent Application No. 14/254,367, filed on April 14, 2014, and entitled "Method and Apparatus of Measuring a Gap Between a First and Second Roll."

Before operation, the drive mechanism 500 may be used in various ways to establish the desired minimum distance, Hg, between the pattern surface 126 of the uncompressed pattern element 122 and the first lip 116 and the second lip 118. In an example scenario, the controller 506 may operate the motor 502 to move the slot die applicator 102 toward the substrate carrier 104, wherein the first lip 116 and second lip 118 are moved into contact with a pattern surface 126. Current supplied to the motor 502 can be monitored by the controller 506 and may be correlated with motor output torque. As such, the motor output torque may be used to provide an indication as to when the first lip 116 and second lip 118 are moved into contact with a pattern surface 126. Subsequently, the controller may operate the motor 502 to move the slot die applicator 102 away from or toward the substrate carrier 104 to establish the desired minimum distance, Hg, as detected based on motor feedback device counts from the motor feedback device 502a and/or the position sensor 508. It is to be appreciated that sensed torque of the motor 502 may be used as a basis to move the first lip 116 and the second lip 118 of the slot die applicator 102 either away from or toward the pattern surface 126 during operation.

As previously mentioned, the drive mechanism 500 may also be used during operation to monitor; establish; maintain; and/or change the desired minimum distance, Hg, between the pattern surface 126 of the uncompressed pattern element 122 and the first lip 116 and the second lip 118 based on various parameters.

In some configurations, the drive mechanism 500 may be manually operated by a user to move the slot die applicator 102 to adjust the desired minimum distance, Hg. In some configurations, the controller 500 may automatically operate the drive mechanism 500 to move the slot die applicator 102 to adjust the desired minimum distance, Hg, based on various sensed operating parameters. For example, as shown in Figure 5, the apparatus may include a sensor 510 that detects the temperature, T, of the slot die applicator 102. In some embodiments, the controller 506 may command the drive mechanism 500 to move the slot die applicator 102 toward or away from the substrate carrier 104 based on the sensed temperature, T. In some embodiments, a change in the temperature, T, may result in a change in the minimum distance, Hg. As such, the drive mechanism 500 may move the first lip 116 and the second lip 118 of the slot die applicator 102 either away from or toward the pattern surface 126 to maintain a desired minimum distance, Hg, between the pattern surface 126 of the pattern element 122 and the first lip 116 and the second lip 118. In some instances, the drive mechanism 500 may move the first lip 116 and the second lip 118 of the slot die applicator 102 away from the pattern surface 126 based on an increase in the temperature, T. In some instances, the drive system 500 may move the first lip 116 and the second lip 118 of the slot die applicator 102 toward the pattern surface 126 based on a decrease in the temperature, T. It is also to be appreciated that the sensed temperature, T, may also be taken into account when establishing the desired minimum distance, Hg, before operation as discussed above.

In another example, as shown in Figure 5, the apparatus 100 may include one or more inspection sensors 512 that may be configured to detect various properties of the advancing substrate 106. In some embodiments, the inspection sensor 512 may be configured to indicate the presence or absence of a tear, hole, splice, and/or contaminants in the substrate 106. In some embodiments, the inspection sensor 512 may be provide or communicate measurements and/or numerical indications of detected positions of components and/or substrates; numerical indications of the positions of components and/or substrates relative to other components and/or substrate; and/or numerical indications of the positions of components and/or substrates relative to another physical or virtual reference. In other embodiments, the inspection sensor 512 may provide or communicate images transferred via a standard protocol such as ftp (File Transfer Protocol), DDE (Dynamic Data Exchange), or OPC (Object Linking and Embedding for Process Control), which are stored in a database or stored in a specified directory on an image server. In some embodiments, the inspection sensors 512 may be configured to create profiles representing surface topographies of the substrate 512.

It is to be appreciated that various different types of inspection sensors 512 may be used to monitor substrates and various components. For example, inspection sensors 512 may be configured as photo-optic sensors that receive either reflected or transmitted light and serve to determine the presence or absence of a specific material; metal-proximity sensors that use electromagnetic to determine the presence or absence of a ferromagnetic material; or capacitive or other proximity sensors using any of a number of varied technologies to determine the presence or absence of materials. Inspection sensors 512 may also be configured as vision systems and other sub-processing devices to perform detection and, in some cases, logic to more accurately determine the status of an inspected product. Particular examples of such inspections sensors 512 may include Cognex Insight, DVT Legend or Keyence smart cameras, component vision systems such as National Instruments PXI or PC based vision system such as Cognex Vision-Pro or any other vision system software which can run on a PC platform. It is to be appreciated that various types of sensors 512 may be used, such as for example, structured light sensors. Examples of such sensors may include a Keyence LJ-V7300; Cognex DS1000; SICK Ranger C; and Keyence LJ-V7000 series including a LJ-V7080 head with a LJ-V7001 controller and Automation Technology C2-2040HS-GigE. Additional examples of sensors 512 methods of operation are described in U.S. Patent Nos. 7,460,250; 7,489,410; and 7,667,857.

Based on the foregoing discussion, it is to be appreciated that the controller 500 may automatically operate the drive mechanism 500 to move the slot die applicator 102 to adjust the desired minimum distance, Hg, based on various parameters detected by the inspection sensor 512. For example, the inspection sensor may be configured to detect or provide an indication of the caliper and/or change in caliper of the substrate 106. Based on the sensed caliper the drive mechanism may move the first lip 116 and the second lip 118 of the slot die applicator 102 either away from or toward the pattern surface 126 to define a minimum distance, Hg, between the pattern surface 126 of the pattern element 122 and the first lip 116 and the second lip 118, wherein the minimum distance, Hg, is less than the sensed caliper of the substrate 106.

With reference to the above description and associated figures, it is to be appreciated that the apparatuses 100 herein may be used to apply adhesive 130 discharged from a slot die applicator 102 to a substrate 106 in a pattern by continuously advancing the substrate in a machine direction past a first lip 116, second lip 118, and slot opening 114 in the slot die applicator 102. The substrate 106 may be engaged with a substrate carrier 104 that may include a base surface 124 and a pattern element 122, wherein the pattern element includes a pattern surface 126. The pattern clement 122 protrudes from the base surface 124 to define a distance, Hp, between the pattern surface 126 and the base surface 124. As previously mentioned, in some embodiments, the substrate carrier may include holes 136 instead of or in combination with base surfaces 126 adjacent the pattern element 122. The substrate carrier 104 is positioned adjacent the slot die applicator 102 to define a minimum distance, Hg, between the pattern surface 126 of the uncompressed pattern element 122 and the first lip 116 and the second lip 118 that is less than the unconstrained caliper, Hs, of the substrate 106. The second surface 110 of the substrate 106 may be advanced past the slot die applicator 102 while the first surface 108 of the substrate 106 is disposed on the substrate carrier 104. And the substrate 106 is intermittently compressed between the slot die applicator 102 and the pattern surface 126 of the pattern element 122 by advancing the pattern element as the pattern surface of the pattern element advances past the first lip 116. the slot opening 114. and the second lip 118 of the slot die applicator 102 while the first surface 108 of the substrate 106 is disposed on the substrate carrier 104.

As previously mentioned, the apparatuses 100 and methods herein may he used to provide for the application of adhesives in patterns to substrates and components during the manufacture of various different products, such as disclosed in for example in U.S. Patent No. 8,186,296 and U.S. Patent Publication Nos. 2014/0148774A1; 2014/0144579A1; 2014/0148323A1; and 2014/0148773A1. For the purposes of a specific illustration, Figure 7 shows one example of a disposable absorbent article 250, such as described in U.S. Patent Publication No. 2008/0132865A1, in the form of a diaper 252 that may be constructed from such substrates and components manipulated during manufacture according to the apparatuses and methods disclosed herein. In particular, Figure 7 is a plan view of one embodiment of a diaper 252 including a chassis 254 shown in a flat, unfolded condition, with the portion of the diaper 252 that faces a wearer oriented towards the viewer. A portion of the chassis structure is cut-away in Figure 7 to more clearly show the construction of and various features that may be included in embodiments of the diaper.

As shown in Figure 7, the diaper 252 includes a chassis 254 having a first ear 256, a second ear 258, a third ear 260, and a fourth ear 262. To provide a frame of reference for the present discussion, the chassis is shown with a longitudinal axis 264 and a lateral axis 266. The chassis 254 is shown as having a first waist region 268, a second waist region 270, and a crotch region 272 disposed intermediate the first and second waist regions.

The periphery of the diaper is defined by a pair of longitudinally extending side edges 274, 276; a first outer edge 278 extending laterally adjacent the first waist region 268; and a second outer edge 280 extending laterally adjacent the second waist region 270. As shown in Figure 7, the chassis 254 includes an inner, body-facing surface 282, and an outer, garment-facing surface 284. A portion of the chassis structure is cut-away in Figure 7 to more clearly show the construction of and various features that may be included in the diaper. As shown in Figure 7, the chassis 254 of the diaper 252 may include an outer covering layer 286 including a topsheet 288 and a backsheet 290. An absorbent core 292 may be disposed between a portion of the topsheet 288 and the backsheet 290. As discussed in more detail below, any one or more of the regions may be stretchable and may include an elastomeric material or laminate as described herein. As such, the diaper 252 may be configured to adapt to a specific wearer's anatomy upon application and to maintain coordination with the wearer's anatomy during wear.

The absorbent article may also include an elastic waist feature 202 shown in Figure 7 in the form of a waist band 294 and may provide improved fit and waste containment. The elastic waist feature 202 may be configured to elastically expand and contract to dynamically fit the wearer's waist. The elastic waist feature 202 can be incorporated into the diaper in accordance with the methods discussed herein and may extend at least longitudinally outwardly from the absorbent core 292 and generally form at least a portion of the first and/or second outer edges 278, 280 of the diaper 252. In addition, the elastic waist feature may extend laterally to include the ears. While the elastic waist feature 202 or any constituent elements thereof may comprise one or more separate elements affixed to the diaper, the elastic waist feature may be constructed as an extension of other elements of the diaper, such as the backsheet 290, the topsheet 288, or both the backsheet and the topsheet. In addition, the elastic waist feature 202 may be disposed on the outer, garment-facing surface 284 of the chassis 240; the inner, body-facing surface 282; or between the inner and outer facing surfaces. The elastic waist feature 202 may he constructed in a number of different configurations including those described in U.S. Patent No. 7,432,413; U.S. Patent Publication No. 2007/0142798; and U.S. Patent Publication No. 2007/0287983.

As shown in Figure 7, the diaper 252 may include leg cuffs 296 that may provide improved containment of liquids and other body exudates. In particular, elastic gasketing leg cuffs can provide a sealing effect around the wearer's thighs to prevent leakage. It is to be appreciated that when the diaper is worn, the leg cuffs may be placed in contact with the wearer's thighs, and the extent of that contact and contact pressure may be determined in part by the orientation of diaper on the body of the wearer. The leg cuffs 296 may be disposed in various ways on the diaper 202.

The diaper 252 may be provided in the form of a pant-type diaper or may alternatively be provided with a re-closable fastening system, which may include fastener elements in various locations to help secure the diaper in position on the wearer. For example, fastener elements may be located on the first and second ears and may be adapted to releasably connect with one or more corresponding fastening elements located in the second waist region. It is to be appreciated that various types of fastening elements may be used with the diaper.

Components of the disposable absorbent article (i.e., diaper, disposable pant, adult incontinence article, sanitary napkin, pantiliner, etc.) described in this specification can at least partially be comprised of biosourced content as described in US 2007/0219521A1 Hird et al published on September 20, 2007, US 2011/0139658A1 Hird et al published on June 16, 2011, US 2011/0139657A1 Hird et al published on June 16, 2011, US 2011/0152812A1 Hird et al published on June 23, 2011, US 2011/0139662A1 Hird et al published on June 16, 2011, and US 2011/0139659A1 Hird et al published on June 16, 2011. These components include, but are not limited to, topsheet nonwovens, backsheet films, backsheet nonwovens, side panel nonwovens, barrier leg cuff nonwovens, super absorbent, nonwoven acquisition layers, core wrap nonwovens, adhesives, fastener hooks, and fastener landing zone nonwovens and film bases.

In at least one exemplary configuration, a disposable absorbent article component comprises a bio-based content value from about 10% to about 100% using ASTM D6866-10. method B, in another embodiment, from about 25% to about 75%, and in yet another embodiment, from about 50% to about 60% using ASTM D6866-10, method B.

In order to apply the methodology of ASTM D6866-10 to determine the bio-based content of any disposable absorbent article component, a representative sample of the disposable absorbent article component must be obtained for testing. In at least one embodiment, the disposable absorbent article component can be ground into particulates less than about 20 mesh using known grinding methods (e.g., Wiley® mill), and a representative sample of suitable mass taken from the randomly mixed particles.

In the context of the previous discussion, the apparatuses 100 and methods herein may be used to provide for the application adhesives in patterns to substrates and components during the manufacture of an absorbent article. For example, adhesives may be applied in various patterns to portions of any of the topsheet, backsheet films, backsheet nonwovens, absorbent core, core encapsulation webs, acquisition layer, surge layer, secondary topsheet layer, leg cuffs, waist feature, ears, and fastening elements during the manufacture of an absorbent article. In some instances, the adhesive may be a different color than that of the substrate. In some applications, the apparatuses and methods herein may be adapted to apply adhesives in absorbent core assembly processes, such as described for example in U.S. Patent Publication Nos. 2006/0021695A1; 2006/0048880A1; 2008/0215166A1; and 2010/0051166A1. In some instances, the apparatuses and methods herein may be configured to apply fluid formulations in the form of wetness indicators, such as disclosed for example in U.S. Patent Publication No. 2011/0137274A1. In yet other instances, the apparatuses and methods herein may be configured to apply fastening adhesives for feminine care articles, including sanitary napkins, panty liners, adult incontinence pads, and the like, such as disclosed for example in European Patent Publication No. EP 0745368A1.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

**Claims**

1. A method for applying a fluid to a substrate in a pattern, the method comprising the steps of:

   providing a slot die applicator (102) comprising a slot opening (114), a first lip (116), and a second lip (118), the slot opening (114) located between the first lip and the second lip;
   providing a substrate carrier (104) comprising a pattern element (122), wherein the pattern element (122) comprises a pattern surface (126);
   positioning the slot die applicator (102) adjacent the substrate carrier (104) to define a minimum distance, Hg, between the pattern surface (126) of the pattern element (122) and the first lip (116) and the second lip (118);
   advancing the substrate (106) to the substrate carrier (104), the substrate (106) having a first surface (108) disposed opposite of a second surface (110) and an unconstrained caliper, Hs, wherein the unconstrained caliper, Hs, of the substrate (106) is greater than minimum distance, Hg;
   advancing the second surface (110) of the substrate (106) past the slot die applicator (102) while the first surface (108) of the substrate (106) is disposed on the substrate carrier (104);
   discharging fluid (130) from the slot opening (114) of the slot die applicator (102) onto the second surface (110) of the substrate (106) in a pattern area (132) having a shape that corresponds with a shape of the pattern surface (126) on the substrate carrier (104) by advancing the pattern surface (126) of the pattern element (122) past the first lip (116), the slot opening (114), and the second lip (118) of the slot die applicator (102) while the first surface (108) of the substrate (106) is disposed on the substrate carrier (104);

moving the first lip (116) and the second lip (118) of the slot die applicator (102) either away from or toward the pattern surface (126) to maintain the minimum distance, Hg, between the pattern surface (126) of the pattern element (122) and the first lip (116) and the second lip (118); and further comprising the step of sensing a temperature, T, of the slot die applicator (102).

2. The method of claim 1, wherein the step of moving the first lip (116) and the second lip (118) of the slot die applicator (102) further comprises moving the first lip (116) and the second lip (118) of the slot die applicator (102) away from the pattern surface (126) based on an increase in the temperature, T.

3. The method of claim 1, wherein the step of moving the first lip (116) and the second lip (118) of the slot die applicator (102) further comprises moving the first lip (116) and the second lip (118) of the slot die applicator (102) toward the pattern surface (126) based on a decrease in the temperature, T.

4. The method according to any of the preceding claims, further comprising the step of connecting a motor (502) with the slot die applicator (102), wherein the motor is configured to move the slot die applicator (102) to change the minimum distance, Hg, between the pattern surface (126) of the pattern element (122) and the first lip (116) and the second lip (118).

5. The method of claim 4, wherein the step of positioning the slot die applicator (102) adjacent the substrate carrier (104) to define the minimum distance, Hg, further comprises operating the motor to move the first lip (116) and second lip (118) into contact with the pattern surface (126) and subsequently operating the motor (502) to move the first lip (116) and second lip (118) away from the pattern surface (126).

6. The method of claim 5, further comprising the step of sensing motor feedback device counts as the motor (502) moves the first lip (116) and second lip (118) away from the pattern surface (126), and correlating the motor feedback device counts with the minimum distance, Hg.

7. The method of claim 4, further comprising the steps of: sensing torque of the motor (502), and further comprising the step of moving the first lip (116) and the second lip (118) of the slot die applicator (102) either away from or toward the pattern surface (126) based on sensed torque of the motor (502).

8. The method according to any of the preceding claims, further comprising the step of advancing the pattern surface (126) past a sensor (508) to provide a measured distance, Md, between the pattern surface (126) of the pattern element (122) and the first lip (116) and the second lip (118).

9. The method of claim 8, wherein the step of moving the first lip (116) and the second lip (118) of the slot die applicator (102) further comprises comparing the measured distance, Md, to a setpoint.

10. The method of claim 8, wherein the sensor (508) comprises a capacitance probe.

**Patentansprüche**

1. Verfahren zum Auftragen eines Fluids auf ein Substrat in einem Muster, wobei das Verfahren folgende Schritte umfasst:

Bereitstellen eines Schlitzdüsenapplikators (102), der eine Schlitzöffnung (114), eine erste Lippe (116) und eine zweite Lippe (118) umfasst, wobei die Schlitzöffnung (114) zwischen der ersten Lippe und der zweiten Lippe angeordnet ist;
Bereitstellen eines Substratträgers (104), der ein Musterelement (122) umfasst, wobei das Musterelement (122) eine Musterfläche (126) umfasst;
Anordnen des Schlitzdüsenapplikators (102) benachbart zu dem Substratträger (104), um einen Mindestabstand, Hg, zwischen der Musterfläche (126) des Musterelements (122) und der ersten Lippe (116) und der zweiten Lippe (118) zu definieren;
Vorwärtsbewegen des Substrats (106) zum Substratträger (104), wobei das Substrat (106) eine erste Fläche (108), die gegenüber einer zweiten Fläche (110) angeordnet ist, und eine nicht zusammengepresste Dicke, Hs, aufweist, wobei die nicht zusammengepresste Dicke, Hs, des Substrats (106) größer als der Mindestabstand, Hg, ist;
Vorwärtsbewegen der zweiten Fläche (110) des Substrats (106) an dem Schlitzdüsenapplikator (102) vorbei, während die erste Fläche (108) des Substrats (106) auf dem Substratträger (104) angeordnet ist;

Abgeben von Fluid (130) aus der Schlitzöffnung (114) des Schlitzdüsenapplikators (102) auf die zweite Fläche (110) des Substrats (106) in einem Musterbereich (132) mit einer Form, die einer Form der Musterfläche (126) an dem Substratträger (104) entspricht, indem die Musterfläche (126) des Musterelements (122) an der ersten Lippe (116), der Schlitzöffnung (114) und der zweiten Lippe (118) des Schlitzdüsenapplikators (102) vorbei bewegt wird, während die erste Fläche (108) des Substrats (106) auf dem Substratträger (104) angeordnet ist; Bewegen der ersten Lippe (116) und der zweiten Lippe (118) des Schlitzdüsenapplikators (102) entweder weg von der Musterfläche (126) oder zu dieser hin, um den Mindestabstand, Hg, zwischen der Musterfläche (126) des Musterelements (122) und der ersten Lippe (116) und der zweiten Lippe (118) aufrechtzuerhalten; und ferner umfassend den Schritt des Messens einer Temperatur, T, des Schlitzdüsenapplikators (102).

2. Verfahren nach Anspruch 1, wobei der Schritt des Bewegens der ersten Lippe (116) und der zweiten Lippe (118) des Schlitzdüsenapplikators (102) ferner das Bewegen der ersten Lippe (116) und der zweiten Lippe (118) des Schlitzdüsenapplikators (102) weg von der Musterfläche (126) aufgrund einer Zunahme der Temperatur, T, umfasst.

3. Verfahren nach Anspruch 1, wobei der Schritt des Bewegens der ersten Lippe (116) und der zweiten Lippe (118) des Schlitzdüsenapplikators (102) ferner das Bewegen der ersten Lippe (116) und der zweiten Lippe (118) des Schlitzdüsenapplikators (102) zu der Musterfläche (126) hin aufgrund einer Abnahme der Temperatur, T, umfasst.

4. Verfahren nach einem der vorstehenden Ansprüche, ferner umfassend den Schritt des Verbindens eines Motors (502) mit dem Schlitzdüsenapplikator (102), wobei der Motor dazu konfiguriert ist, den Schlitzdüsenapplikator (102) zu bewegen, um den Mindestabstand, Hg, zwischen der Musterfläche (126) des Musterelements (122) und der ersten Lippe (116) und der zweiten Lippe (118) zu ändern.

5. Verfahren nach Anspruch 4, wobei der Schritt des Anordnens des Schlitzdüsenapplikators (102) benachbart zu dem Substratträger (104), um den Mindestabstand, Hg, zu definieren, ferner das Betreiben des Motors, um die erste Lippe (116) und die zweite Lippe (118) in Kontakt mit der Musterfläche (126) zu bewegen, und das anschließende Betreiben des Motors (502) umfasst, um die erste Lippe (116) und die zweite Lippe (118) von der Musterfläche (126) weg zu bewegen.

6. Verfahren nach Anspruch 5, ferner umfassend den Schritt des Messens von Zählwerten einer Motorrückkopplungsvorrichtung, während der Motor (502) die erste Lippe (116) und die zweite Lippe (118) von der Musterfläche (126) weg bewegt, und des Korrelierens der Zählwerte der Motorrückkopplungsvorrichtung mit dem Mindestabstand, Hg.

7. Verfahren nach Anspruch 4, ferner folgende Schritte umfassend: Messen von Drehmoment des Motors (502), und ferner umfassend den Schritt des Bewegens der ersten Lippe (116) und der zweiten Lippe (118) des Schlitzdüsenapplikators (102) entweder weg von der Musterfläche (126) oder zu dieser hin aufgrund des gemessenen Drehmoments des Motors (502).

8. Verfahren nach einem der vorstehenden Ansprüche, ferner umfassend den Schritt des Vorwärtsbewegens der Musterfläche (126) an einem Sensor (508) vorbei, um einen gemessenen Abstand, Md, zwischen der Musterfläche (126) des Musterelements (122) und der ersten Lippe (116) und der zweiten Lippe (118) bereitzustellen.

9. Verfahren nach Anspruch 8, wobei der Schritt des Bewegens der ersten Lippe (116) und der zweiten Lippe (118) des Schlitzdüsenapplikators (102) ferner das Vergleichen des gemessenen Abstands, Md, mit einem Sollwert umfasst.

10. Verfahren nach Anspruch 8, wobei der Sensor (508) eine Kapazitanzsonde umfasst.

## Revendications

1. Procédé d'application d'un fluide sur un substrat dans un motif, le procédé comprenant les étapes consistant à :

fournir un applicateur à filière plate (102) comprenant une ouverture de fente (114), une première lèvre (116) et une deuxième lèvre (118), l'ouverture de fente (114) étant située entre la première lèvre et la deuxième lèvre ; fournir un support de substrat (104) comprenant un élément de motif (122), dans lequel l'élément de motif (122) comprend une surface de motif (126) ; positionner l'applicateur à filière plate (102) adjacent au support de substrat (104) pour définir une distance minimale, Hg, entre la surface de motif (126) de l'élément de motif (122) et la première lèvre (116) et la deuxième lèvre (118) ; faire avancer le substrat (106) vers le support de substrat (104), le substrat (106) ayant une première surface (108) disposée opposée à une deuxième surface (110) et un calibre sans contrainte, Hs, dans lequel le calibre sans contrainte, Hs, du substrat (106) est supérieur à la distance minimale, Hg ;

faire avancer la deuxième surface (110) du substrat (106) au-delà de l'applicateur à filière plate (102) alors que la première surface (108) du substrat (106) est disposée sur le support de substrat (104) ;

décharger un fluide (130) depuis l'ouverture de fente (114) de l'applicateur à filière plate (102) sur la deuxième surface (110) du substrat (106) dans une zone de motif (132) ayant une forme qui correspond à une forme de la surface de motif (126) sur le support de substrat (104) en faisant avancer la surface de motif (126) de l'élément de motif (122) au-delà de la première lèvre (116), de l'ouverture de fente (114) et de la deuxième lèvre (118) de l'applicateur à filière plate (102) alors que la première surface (108) du substrat (106) est disposée sur le support de substrat (104) ;

déplacer la première lèvre (116) et la deuxième lèvre (118) de l'applicateur à filière plate (102) soit à l'écart soit en direction de la surface de motif (126) pour maintenir la distance minimale, Hg, entre la surface de motif (126) de l'élément de motif (122) et la première lèvre (116) et la deuxième lèvre (118) ; et comprenant en outre l'étape de détection d'une température, T, de l'applicateur à filière plate (102).

2. Procédé selon la revendication 1, dans lequel l'étape de déplacement de la première lèvre (116) et de la deuxième lèvre (118) de l'applicateur à filière plate (102) comprend en outre le déplacement de la première lèvre (116) et de la deuxième lèvre (118) de l'applicateur à filière plate (102) à l'écart de la surface de motif (126) sur la base d'une augmentation de la température, T.

3. Procédé selon la revendication 1, dans lequel l'étape de déplacement de la première lèvre (116) et de la deuxième lèvre (118) de l'applicateur à filière plate (102) comprend en outre le déplacement de la première lèvre (116) et de la deuxième lèvre (118) de l'applicateur à filière plate (102) en direction de la surface de motif (126) sur la base d'une diminution de la température, T.

4. Procédé selon l'une quelconque des revendications précédentes, comprenant en outre l'étape de raccordement d'un moteur (502) à l'applicateur à filière plate (102), dans lequel le moteur est configuré pour déplacer l'applicateur à filière plate (102) pour changer la distance minimale, Hg, entre la surface de motif (126) de l'élément de motif (122) et la première lèvre (116) et la deuxième lèvre (118).

5. Procédé selon la revendication 4, dans lequel l'étape de positionnement de l'applicateur à filière plate (102) adjacent au support de substrat (104) pour définir la distance minimale, Hg, comprend en outre la mise en oeuvre du moteur pour déplacer la première lèvre (116) et la deuxième lèvre (118) en contact avec la surface de motif (126) et la mise en oeuvre ultérieure du moteur (502) pour déplacer la première lèvre (116) et la deuxième lèvre (118) à l'écart de la surface de motif (126).

6. Procédé selon la revendication 5, comprenant en outre l'étape de détection de comptages d'un dispositif de rétroaction du moteur à mesure que le moteur (502) déplace la première lèvre (116) et la deuxième lèvre (118) à l'écart de la surface de motif (126), et la corrélation des comptages du dispositif de rétroaction du moteur avec la distance minimale, Hg.

7. Procédé selon la revendication 4, comprenant en outre les étapes consistant à : détecter le couple du moteur (502), et comprenant en outre l'étape de déplacement de la première lèvre (116) et de la deuxième lèvre (118) de l'applicateur à filière plate (102) soit à l'écart soit en direction de la surface de motif (126) sur la base du couple détecté du moteur (502).

8. Procédé selon l'une quelconque des revendications précédentes, comprenant en outre l'étape consistant à faire avancer la surface de motif (126) au-delà d'un capteur (508) pour fournir une distance mesurée, Md, entre la surface de motif (126) de l'élément de motif (122) et la première lèvre (116) et la deuxième lèvre (118).

9. Procédé selon la revendication 8, dans lequel l'étape de déplacement de la première lèvre (116) et de la deuxième lèvre (118) de l'applicateur à filière plate (102) comprend en outre la comparaison de la distance mesurée, Md, à un point de consigne.

10. Procédé selon la revendication 8, dans lequel le capteur (508) comprend une sonde à capacité.

Fig. 1

Fig. 2A

Fig. 2B

Fig. 3A

EP 3 151 798 B1

Fig. 3B

Fig. 2C

Fig. 3C

Fig. 4

Fig. 4A1

Fig. 4A2

Fig. 5

EP 3 151 798 B1

Fig. 6A

Fig. 6B

Fig. 6C

Fig. 6D

Fig. 6E

Fig. 7

**REFERENCES CITED IN THE DESCRIPTION**

**Patent documents cited in the description**

- US 2011274834 A
- US 8186296 B
- US 20140148774 A1
- US 20140144579 A1
- US 20140148323 A1
- US 20140148773 A1
- US 7056386 B
- US 5286543 A
- US 5359525 A
- US 6801828 B
- US 6820022 B
- US 7123981 B
- US 8145343 B
- US 8145344 B
- US 8244393 B
- EP 1528907 B1
- US 25436714 A
- US 7460250 B
- US 7489410 B
- US 7667857 B
- US 20080132865 A1
- US 7432413 B
- US 20070142798 A
- US 20070287983 A
- US 20070219521 A1, Hird
- US 20110139658 A1, Hird
- US 20110139657 A1, Hird
- US 20110152812 A1, Hird
- US 20110139662 A1, Hird
- US 20110139659 A1, Hird
- US 20060021695 A1
- US 20060048880 A1
- US 20080215166 A1
- US 20100051166 A1
- US 20110137274 A1
- EP 0745368 A1